# EUROPEAN PATENT SPECIFICATION

(11) **EP 4 230 032 B1**
(45) Date of publication and mention of the grant of the patent: **01.07.2026**
(21) Application number: 22157224.1
(22) Date of filing: 17.02.2022
(51) Int. Cl.: A61B 6/50, A01K 1/06, A61B 6/03, A61B 6/04

(54) **MULTIMODALITY FOLDABLE MULTIPLE SMALL ANIMALS HOLDER**
MULTIMODALER FALTBARER HALTER FÜR MEHRERE KLEINE TIERE
HARNAIS POUR PLUSIEURS PETITS ANIMAUX PLIABLE À MULTIMODALITÉ

(43) Date of publication of application: 23.08.2023
(73) Proprietor: Bruker Belgium N.V., 2550 Kontich (BE)
(72) Inventor: Claessens, Timothy, 3270 Scherpenheuvel (BE)
(74) Representative: Kohler Schmid Möbus Patentanwälte

(56) References cited:
- WO-A2-2013/036597
- US-B2- 8 660 633

## Description

The invention relates to a holding device for simultaneously holding several small animals comprising at least three beds each for a small animal, whereby each bed is attached to an according holder element.

A holding device with these features is known from WO 2013/036597 A2 (= Reference [0]).

Holding devices for simultaneous holding a multitude of beds with small animals thereon are for example described in US 8,660,633 B2 (= Reference [1]) or in Esparza-Cross 2008: "Wireless self-gated multiple-mouse cardiac cine MRI", Mag. Resonance in Medicine 59, 1203-1206 (2008) (=Reference [2]).

### Technical background of the invention

In general, the present invention relates to the technical field of laboratory support for conducting scientific investigations on - in most cases living - animals. In particular, the invention contributes as an auxiliary aid for holding small animals, like e.g. mice and rats, during examinations.

US 10,632,271 B1 (=Reference [3]) describes a "versatile subject bed" for use with one or more test subjects that is configured to keep the subjects sedated throughout either a surgical procedure or imaging process. This subject bed can include interchangeable manifolds allowing the platform to hold up to four subjects at the same time. The four-subject manifold can include an elevated platform that holds two subject side by side and allows the subjects to be stacked during imaging or other procedures.

In Reference [2] cited above, four mice can be inserted geometrically in parallel into an MRI imaging device. However, these mouse holders are mechanically separated from each other.

Reference [1] also cited above describes a generic holding device for simultaneously holding several small animals. Here, each small animal can be separately fixed to a bed and inserted into a common holder which provides anesthesia ports. The animal beds can all be attached to the holder, but must be inserted separately into the holder. The animal beds can be separated from the holder for individually placing the small animals. After mounting, the beds each have a fixed position relative to the holder and to each other. In particular, the device according to Reference [1] does not enable any possibility of moving the beds with respect to each other.

Although there is a wide range of already existing solutions according to prior art devices for holding a multitude of small animals at the same time, no one of them offers the combination of a compact device and an easy animal access when mounted to the holder.

### Objects of the invention

It is an object of the present invention to provide -without much technical efforta generic and compact holding device for a multitude of small animals facilitating a flexible, easy access to each small animal when attached to the holder, in particular for generating a 3D image of all animals at the same time, e.g. in an MRI, PET, CT scanner or the like. The holding system should be easy to use, in particular for rats or mice, and easily to be applied in a wide variety of locations through its small and compact construction.

### Brief description of the invention

This object is achieved, in accordance with the present invention and in a surprisingly simple and effective way, by modifying a generic device for simultaneously holding several small animals as defined above in the first paragraph in that the holding device is geometrically constructed and mechanically configured such that two holder elements can be moved together with their respective beds above a third holder element without thereby changing the vertical orientation of the beds being attached to the two holder elements.

The holding device according to the invention is geometrically constructed and configured such that the two holder elements can be moved above the third holder element by a translatory movement or alternatively by a rotating movement.

The holding device according to the invention is designed to be stable due to the translatory adjustability of the holding elements and is a compact arrangement of the animals' beds. Small animals are kept in horizontal position once the beds/holders are arranged for cross-sectional scanning i.e. tomographic modalities. Only that allows for arranging the small animals like that. For surface scanning (optical, thermography) they need to be arranged next to each other.

However, in some other cases of application, there might be some need to avoid translatory movement. For these cases, alternative variants of the invention are suitable, where the two holder elements can be moved above the third holder element by a rotating movement.

A rotating movement allows a compact setup of the holding device and reduces the footprint in the unfolded position. This might apply if there is no need to keep the beds attached to the holder horizontal, e.g. when used in a scanner for material samples, e.g. bore cores.

The holding device according to the invention is adapted for use as a multiplepurpose animal bed to be able to measure/scan multiple small animals in multiple modalities:
- It can receive e.g. 4 mice or maybe only 2 mice and a rat or larger mouse.
- It can be used in stretched/unfolded mode with e.g. 4 mice near each other, being arranged horizontally or rather in folded mode with e.g. 4 mice arranged in a circle.
- In stretched/unfolded mode, the beds are aligned in a common plane and the bottom surfaces as well.
- Both modes can be mechanically locked.
- While unfolding or folding the bed holder, the orientation of the beds is always in the same vertical direction; therefore, no mouse will accidentally fall off.
- The unfolding mechanism provides enough space for anesthesia supply.

Although the holding device according to the present invention is preferably used in devices for preclinical imaging, there are also other possible and useful implementations of the invention in further fields of research.

### Preferred embodiments and further developments of the invention

In particularly preferred embodiments of the invention, the two holder elements which can be moved above the third holder element are pivotably linked to the third holder element each with at least two holder arms.

By using two holder arms, the mechanical stability during rotation and in the static case is improved.

In another class of advantageous embodiments of the present invention, the holding device has a locking mechanism which is geometrically constructed and mechanically configured such that two of the holder elements can be locked in their movement and fixed in a folded position with at least one of the first two holder elements being placed above the third holder element and in an unfolded position with at least one of the first two holder elements being placed horizontally beside the third holder element.

This mechanism makes the holder mechanically stable in both, the open and closed state, and a small animal can be easily removed without removing a bed from the holder. It prevents the assembly of moving unintendedly during a cross-sectional scanning - maybe induced by an animal moving. Further, this makes sure that for animal preparation - in unfolded position - the assembly does not fold and can be put on a flat surface without toppling over.

In a preferred further development of this embodiment, the holder arms of the two holder elements have deepenings and the third holder element has spring loaded balls which can engage in the deepenings so that the two holder elements can be fixed with respect to the third holder element in the folded position or in the unfolded position.

As a result, the fixation of the two retaining elements in the unfolded and folded positions can be achieved without much force and with a smooth transition. It is the least space consuming way of providing a locking mechanism. Only a single spring loaded ball is needed to lock a holder element into folded and unfolded position.

A preferred variant of this further development is characterized in that the deepenings have a slot-like structure.

This allows for relaxed requirements on manufacturing tolerances of the holding device, as the deepenings of the holder arms do not need to be aligned exactly with the spring loaded balls when being fixed in the folded or unfolded position. Compared to a circular deepening a slot-like deepening allows more freedom of the ball of the spring loaded ball to snap into. In that sense, it allows to compensate for manufacturing tolerances.

Alternatively or conjointly, in a further advantageous variant, the third holder element comprises two spring loaded balls, which engage in the deepening of the upper holder arms and the lower holder arms in the unfolded and in the folded position, respectively.

This reduces material usage as only two spring loaded balls are required for the device to lock into folded and unfolded position, which means less material costs and less mechanical complexity.

A further embodiment of the invention is characterized in that the first and second holder element each have a flat surface at their bottom such that the first and second holder element can stand stable on a flat working surface, in particular on a desk, in an unfolded position with at least one of the first and second holder element being placed horizontally beside the third holder element.

Due to this measure, the device is stable when open and small animals can be removed or inserted freehand. As explained above, this arrangement will make sure that for animal preparation - in unfolded position - the assembly does not fold and can be put on a flat surface without toppling over.

Also favorable are embodiments of the invention, which are characterized in that in a completely folded position, with the first and the second holder element being both placed above the third holder element, the holding device forms a cylindrical shape with circular circumference.

Due to the circular cylinder shape, the device is designed to be particularly compact. A cylindrical shape is especially helpful for cross-sectional imaging.

Preferred is a further development of these embodiments, in which the holder device is constructed in such a way that its cylindrical circumference in the completely folded state fits into an entry opening of an imaging instrument with circular inner diameter.

This would be e.g. a diameter of around 80 mm for an in-vivo micro-CT device or 85 - 200 mm for a magnetic resonance imaging instrument. It is an important requirement that the device needs to fit into the bore for imaging.

In some embodiments, the third holder element comprises two beds for small animals, in particular mice.

This allows to maximize the number of animals to be arranged occupying the least amount of space. It is required for an application which demands a total of 4 beds to be arranged for simultaneous scanning.

In alternative embodiments, the third holder element comprises one bed for a small animal, in particular a rat.

This makes the holding device more flexible to accommodate one smaller and one larger animal and is required to provide enough space even for a larger animal.

In another class of comparatively compact and simple embodiments of the invention, each of the animal beds have a semicircular cross section.

That feature provides a stable holding of each small animal. Further, a semicircular cross section provides enough space for the small animal. In principle, other cross-sections are also possible, but a semicircular cross section is easy to handle and clean since there are no corners compared to rectangular cross sections. In addition, a semicircular cross-section will provide enough space to put a warming pad below the small animal.

Further beneficial embodiments of the invention are characterized in that the beds at their free ends adjacent to their respective holder elements have a holding bar or stopper element preventing the small animal from slipping out of its bed.

The small animals are thereby kept in position even if the holding device is tilted when transferring it e.g. to an imaging instrument. Further, this prevents fluids (e.g. mouse urine) drop out of the bed and pollute the imaging system.

Preferred is a further development of these embodiments, in which the holding bars or stopper elements have a notch for holding a tail of the small animal while lying in the bed.

In this way, the small animals are at least partially fixed in an upward or downward position. In addition, this might act as a defined outlet for fluids.

Still other advantageous embodiments of the holding device according to the present invention are characterized in that each of the holder elements have a slot or slit for receiving and holding inlet tubes for leading anesthetization gas to a gas mask for the respective small animal.

This arrangement allows for a compact design of the entire assembly, since no inlet tubes need to be routed outside the perimeter of the holding device. It can be combined with an afore mentioned anesthesia mask.

Further advantages can be extracted from the description and the enclosed drawings. The features mentioned above and below can be used in accordance with the invention either individually or collectively in any combination. The embodiments mentioned are not to be understood as exhaustive enumeration but rather have exemplary character for the description of the invention.

### Detailed description of the invention and drawings

The invention is shown in the drawings and is explained in more detail based on illustrative embodiments.

In the drawings:
- Fig. 1a: shows a stereoscopic view of an embodiment of the holding device according to the invention in a folded position of the holder elements and four beds attached thereto with a mouse conveniently lying on one of the upper beds having its tail hanging over a notch of a stopper element;
- Fig. 1b: shows a schematic horizontal front view on the holding device as shown in Fig. 1a in direction looking from the beds to the holder elements;
- Fig. 2a: shows in a stereoscopic view the embodiment of Fig. 1a in an unfolded position with the attached beds arranged side by side in a horizontal plane;
- Fig. 2b: shows a schematic horizontal front view on the holding device in its unfolded position as shown in Fig. 2a looking in direction from the beds to the holder elements;
- Fig. 3a: shows a schematic vertical cross section through a holding device in the unfolded position as shown in Fig. 2a, displaying holder arms pivotably linking the holder elements;
- Fig. 3b: shows a schematic vertical cross section through a holding device as shown in Fig. 3a, however in a partially folded position;
- Fig. 3c: shows a schematic horizontal front view on a holding device as shown in Fig. 3a in its completely folded position looking in direction from the beds to the holder elements, the holding device having only three beds, namely two smaller beds in the upper position and a bigger bed in the lower position;
- Fig. 4a: shows a schematic stereoscopic view of an embodiment of the holding device according to the invention having four beds and being in a partially folded position;
- Fig. 4b: shows an enhanced part of the embodiment of Fig. 4a displaying screws with spring loaded balls engaged in according deepenings;
- Fig. 4c: shows a schematic stereoscopic view of an embodiment of the invention similar to the holding device as shown in Fig. 4a in a partially folded position; and
- Fig. 4d: shows a schematic, semi-transparent horizontal front view on the holding device as shown in Fig. 4c in a partially folded position displaying holder arms pivotably linking the holder elements.

The present invention is principally concerned with providing tools for performing scientific investigations on small animals, in particular with the development of an improved holding device for a multitude of small animals like mice and rats.

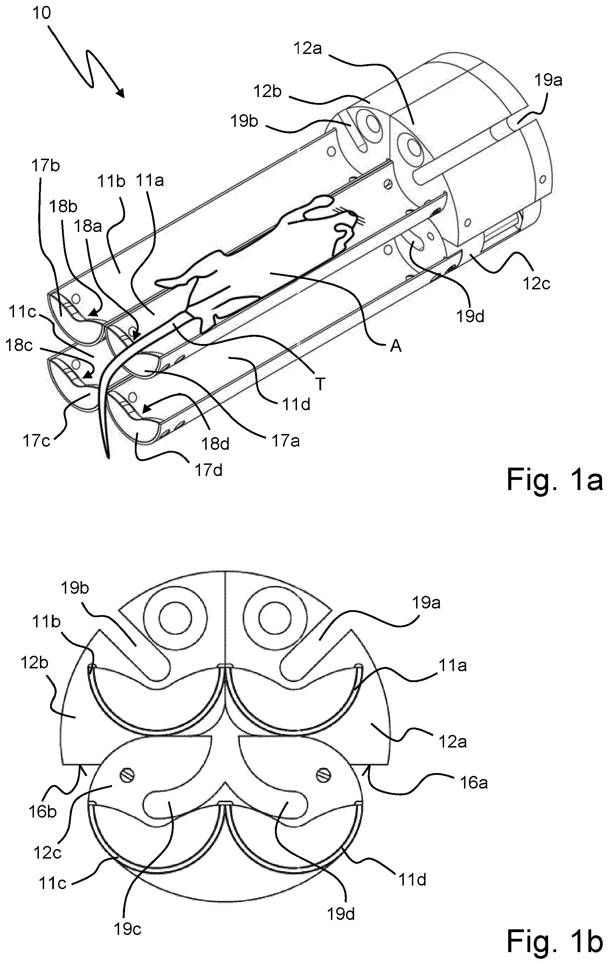

**Fig. 1a** schematically depicts a **holding device 10** for simultaneously holding several **small animals A** comprising at least three **beds 11a, 11b, 11c, 11d** each for a small animal A, whereby each bed 11a, 11b, 11c, 11d is attached to an according **holder element 12a, 12b, 12c.**

According to the invention, this holding device 10 is geometrically constructed and mechanically configured such that two of the holder elements 12a, 12b can be moved together with their respective beds 11a, 11b above a third holder element 12c without thereby changing the vertical orientation of the beds 11a, 11b being attached to the two holder elements 12a, 12b.

The holding device 10 of Fig. 1a is depicted in a folded position of the holder elements 12a, 12b, 12c with two beds 11a, 11b attached to the two holder elements 12a, 12b in the upper position and another two beds 11c, 11d attached to the third holder element 12c in the lower position. The two holder elements 12a, 12b can be moved above the third holder element 12c by a rotating movement in this embodiment.

The beds 11a, 11b, 11c, 11d in this embodiment each have a semicircular cross section. In a completely folded position with the first and the second holder element 12a, 12b being both placed above the third holder element 12c the holding device 10 forms a cylindrical shape with circular circumference. This provides the biggest amount of animals in a circle and in this way one can easily use circular scan methods like CT, PET etc..

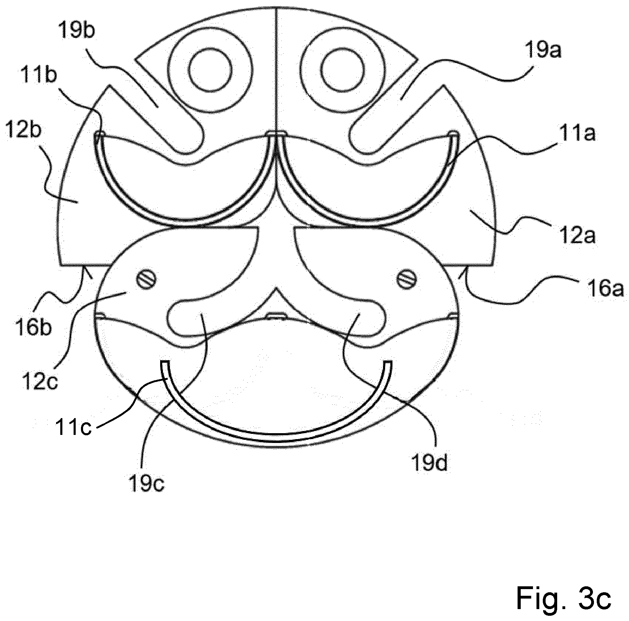

The third holder element 12c in this embodiment comprises two beds 11c, 11d for small animals A, in particular mice. In other embodiments of the invention (as shown in Fig. 3c), the third holder element 12c comprises only one bed 11c for a small animal A, in particular a bigger mouse or a rat.

Further, **holding bars or stopper elements 17a, 17b, 17c, 17d** are provided, each having a **notch 18a, 18b, 18c, 18d** for holding a **tail T** of the small animal A while lying on its bed 11a, 11b, 11c, 11d. The holder elements 12a, 12b, 12c each have a **slot or slit 19a, 19b, 19c, 19d** for receiving and holding inlet tubes for leading anesthetization gas to a gas mask for the respective small animal A.

**Fig. 1b** shows a schematic horizontal front view on the holding device as shown in Fig. 1a in direction looking from the beds 11a, 11b, 11c, 11d to the holder elements 12a, 12b, 12c.

The first and second holder element 12a, 12b each have a **flat surface 16a, 16b** at their bottom such that the first and second holder element 12a, 12b can stand stable on a flat working surface, in particular on a desk, in an unfolded position with at least one of the first and second holder element 12a, 12b being placed horizontally beside the third holder element 12c.

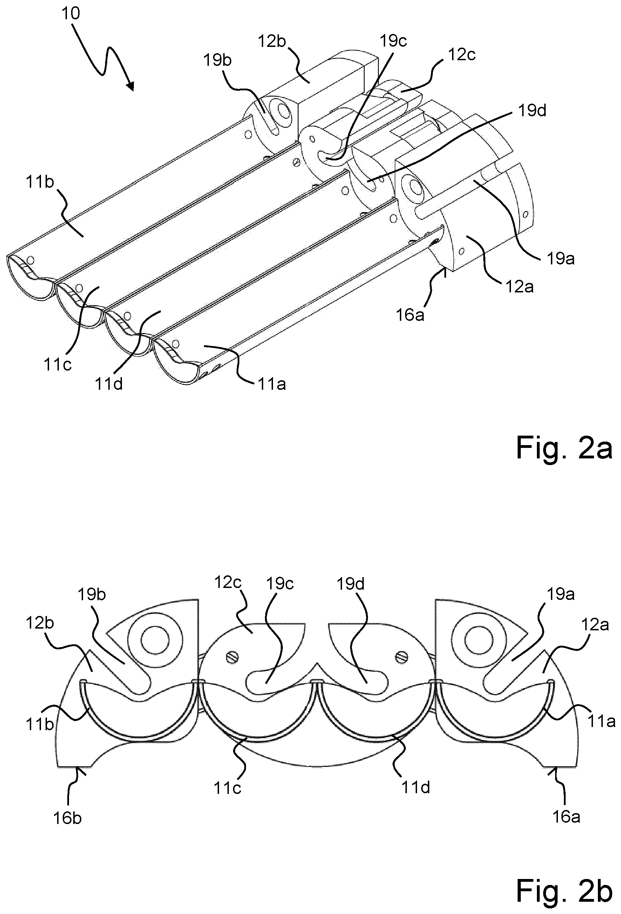

**Fig. 2a** shows in a stereoscopic view the holding device 10 of Fig. 1a in the unfolded position with the attached beds 11a, 11b, 11c, 11d arranged side by side in a horizontal plane. In this unfolded, stretched position, up to four small animals can be easily arranged in the holding device 10 or removed therefrom after measurement. In this flat setup, one can easily prepare four mice or scan in an optical scanner, if 2D images are needed and one cannot scan through multiple mice at the same time.

**Fig. 2b** shows in a schematic horizontal front view the holding device 10 in its unfolded position as depicted in Fig. 2a, looking again in a direction from the beds 11a, 11b, 11c, 11d to the holder elements 12a, 12b, 12c. Flat surfaces 16a, 16b are aligned with lower portion of the third holder element 12c to ensure a stable positioning of the holder on a flat surface.

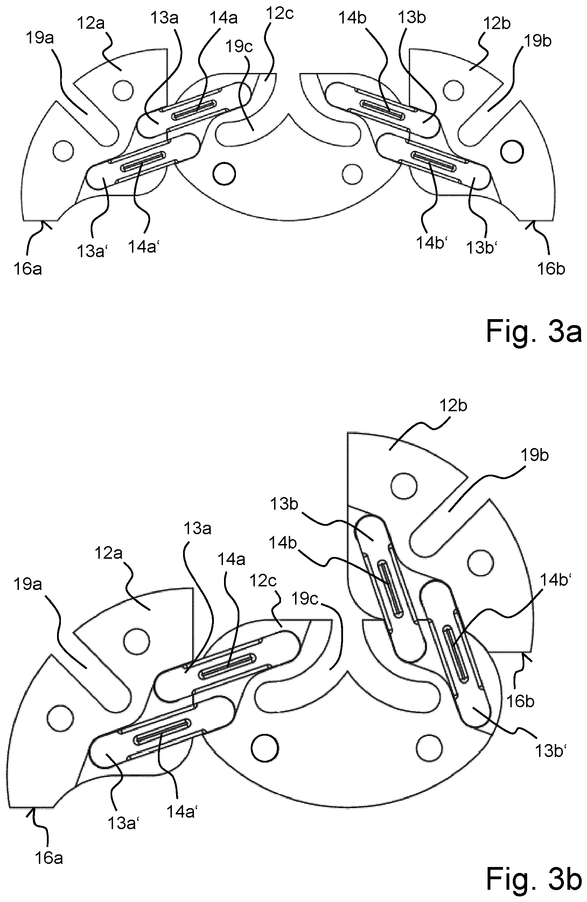

**Fig. 3a** displays a schematic vertical cross section through a holding device 10 in its unfolded position as shown in Fig. 2b. The two holder elements 12a, 12b can again be rotationally moved above the third holder element 12c and are pivotably linked to the third holder element 12c each with at least two **holder arms 13a,13a'; 13b, 13b'.** These holder arms make sure that the folding movement is made in such a way that the orientation of the beds is always vertical.

The holding device 10 has a locking mechanism which is geometrically constructed and mechanically configured such that two of the holder elements 12a, 12b in their movement can be locked and fixed in a folded position with at least one of the first and second holder element 12a, 12b being placed above the third holder element 12c and in an unfolded position with at least one of the first and second holder element 12a, 12b being placed horizontally beside the third holder element 12c.

The holder arms 13a, 13a'; 13b,13b' of the two holder elements 12a, 12b have **deepenings 14a,14a'; 14b,14b'** with a slot-like structure.

**Fig. 3b** shows a schematic vertical cross section through a holding device 10 as shown in Fig. 3a, however in a partially folded position of the holder element 12b on the right side. Holder arms 13a,13a'; 13b, 13b' are attached to the respective holder elements 12a, 12b and 12c such that they remain parallel during and after the rotary movement.

**Fig. 3c** shows a schematic horizontal front view on a holding device 10 as shown in Fig. 3a in its completely folded position looking in direction from the beds 11a, 11b, 11c to the holder elements 12a, 12b, 12c. In this embodiment, the holding device 10 has only three beds 11a, 11b, 11c, namely two smaller beds 11a, 11b in the upper position and a bigger bed 11c in the lower position. Now, the small animals are arranged over 120 degree instead off 90 degree like it is with the 4-mice-formation as e.g. shown in Figs. 1a and 1b.

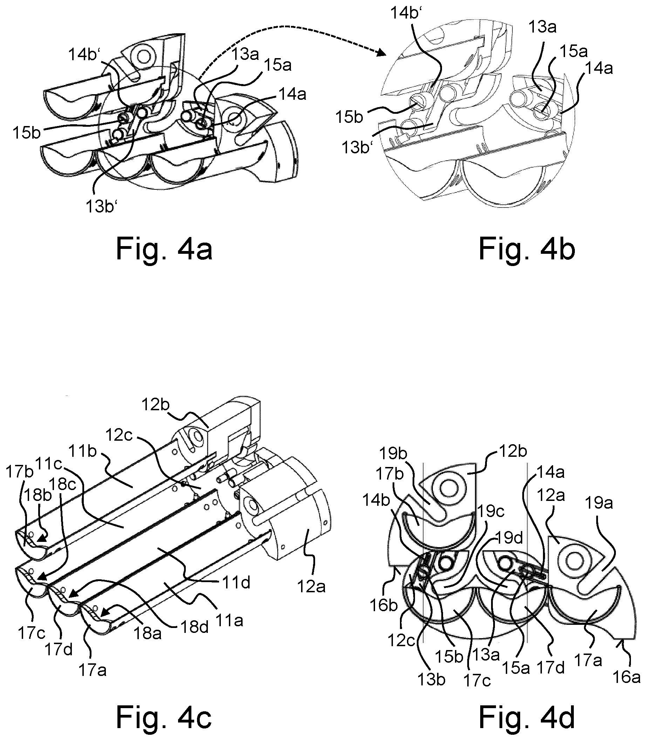

A schematic stereoscopic view of an embodiment of the holding device according to the invention having four beds and being in a partially folded position with one of the beds in the lifted position and the other three beds in the lower horizontal arrangement is depicted in **Fig. 4a****.**

The third holder element 12c carrying two beds 11c, 11d comprises **screws with spring loaded balls 15a, 15b** which can engage in the deepenings 14a, 14b' of the holder arms 13a, 13b' so that the first two holder elements 12a, 12b can be fixed with respect to the third holder element 12c in the folded position or in the unfolded (stretched) position. In Figures 4a - 4d, a portion of holder element 12c has been omitted to better reveal the location and function of the screws with spring loaded balls 15a, 15b and the deepenings 14a, 14b' of the respective holder arms.

**Fig. 4b** shows an enhanced part of the embodiment of Fig. 4a displaying the screws with spring loaded balls 15a, 15b engaged in according deepenings 14a, 14b'.

More precisely, Fig. 4b merely depicts an adjustable frame for the spring loaded balls, i.e. the screws 15a, 15b, the former not being visible in this picture. In this embodiment, the spring pressure can be adjusted by means of a screwdriver (not displayed here) or the like. Figures 2b, 3c and show an opening for accessing these screws from the front of the holder. This allows easy adjustment of the holding pressure of the spring loaded balls.

The third (bottom) holder element 12c contains two ball screws, which are inserted from the backside. Once the holder arm 13b' moves over the spring loaded ball screw, the ball snaps into the grove 14b' and will lock the movement of the arm 13b'. That means, the spring loaded ball screws 15a, 15b are not part of the holder arms, but part of and rigidly connected to the third (bottom) holder element 12c. In fact, there is a little hole in the third (bottom) holder element 12c on the front (bed) side that allows to adjust the fit of the ball screw and thus of the load onto the holder arm. In the figure, the third (bottom) holder element is hidden and reveals the inserted ball screws and it looks like as they are connected to the arms - which they are not in fact. But the figures nicely illustrate that the ball snaps into the grove of the top holder arm in case the assembly is open and into to bottom holder arms once it is closed.

The stereoscopic view of **Fig. 4c** shows an embodiment of the invention similar to the holding device as shown in Fig. 4a in the same partially folded position.

Finally, **Fig. 4d** shows a schematic, semi-transparent front view on the holding device as shown in Fig. 4c in its partially folded position displaying holder arms 13a, 13b pivotably linking the first holder elements 12a, 12b to the third holder element 12c.

### List of reference signs:

- 10: holding device
- 11a, 11b, 11c, 11d: beds
- 12a, 12b: first and second holder elements
- 12c: third holder element
- 13a; 13b: upper holder arms
- 13a'; 13b': lower holder arms
- 14a,14a'; 14b,14b': deepenings
- 15a, 15b: screws with spring loaded balls
- 16a, 16b: flat surfaces
- 17a, 17b, 17c, 17d: holding bars or stopper elements
- 18a, 18b, 18c, 18d: notches
- 19a, 19b, 19c, 19d: slots or slits
- A: small animal
- T: tail

### Prior art citations:

Publications considered for assessing patentability of the present invention:
[0] WO 2013/036597 A2
[1] US 8,660,633 B2
[2] Esparza-Cross 2008: "Wireless self-gated multiple-mouse cardiac cine MRI", Mag. Resonance in Medicine 59, 1203-1206 (2008)
[3] US 10,632,271 B1

## Claims

1. Holding device (10) for simultaneously holding several small animals (A) comprising
at least three beds (11a, 11b, 11c, 11d) each for a small animal (A), whereby each bed (11a, 11b, 11c, 11d) is attached to an according holder element (12a, 12b, 12c),
**characterized**
**in that** the holding device (10) is geometrically constructed and mechanically configured such that two of the holder elements (12a, 12b) can be moved together with their respective beds (11a, 11b) above a third holder element (12c) without thereby changing the vertical orientation of the beds (11a, 11b) being attached to the two holder elements (12a, 12b), and
**in that** the two holder elements (12a, 12b) can be moved above the third holder element (12c) by a translatory movement or by a rotating movement.

2. Holding device according to claim 1, **characterized in that** the two holder elements (12a, 12b) which can be moved above the third holder element (12c) are pivotably linked to the third holder element (12c) each with at least two holder arms (13a,13a'; 13b,13b').

3. Holding device according to anyone of the preceding claims, **characterized in that** the holding device (10) has a locking mechanism which is geometrically constructed and mechanically configured such that two of the holder elements (12a, 12b) in their movement can be locked and fixed in a folded position with at least one of the first and second holder element (12a, 12b) being placed above the third holder element (12c) and in an unfolded position with at least one of the first and second holder element (12a, 12b) being placed horizontally beside the third holder element (12c).

4. Holding device according to claims 2 and 3, **characterized in that** the holder arms (13a, 13a'; 13b, 13b') of the two holder elements (12a, 12b) have deepenings (14a,14a'; 14b,14b') and the third holder element (12c) has spring loaded balls (15a, 15b) which can engage in the deepenings (14a,14a'; 14b,14b') so that the two holder elements (12a, 12b) can be fixed with respect to the third holder element (12c) in the folded position or in the unfolded position.

5. Holding device according to claim 4, **characterized in that** the deepenings (14a,14a'; 14b,14b') have a slot-like structure.

6. Holding device according to claim 4 or 5, **characterized in that** the third holder element (12c) comprises two spring loaded balls (15a, 15b) which engage in the deepening of the upper holder arms (13a, 13b) and the lower holder arms (13a', 13b') in the unfolded and in the folded position, respectively.

7. Holding device according to anyone of the preceding claims, **characterized in that** the first and second holder element (12a, 12b) each have a flat surface (16a, 16b) at their bottom such that the first and second holder element (12a, 12b) can stand stable on a flat working surface, in particular on a desk, in an unfolded position with at least one of the first and second holder element (12a, 12b) being placed horizontally beside the third holder element (12c).

8. Holding device according to anyone of the preceding claims, **characterized in that** in a completely folded position with the first and the second holder element (12a, 12b) being both placed above the third holder element (12c) the holding device (10) forms a cylindrical shape with circular circumference.

9. Holding device according to anyone of claims 1 through 8, **characterized in that** the third holder element (12c) comprises two beds (11c, 11d) for small animals (A), in particular mice.

10. Holding device according to anyone of claims 1 through 8, **characterized in that** the third holder element (12c) comprises one bed (11c) for a small animal (A), in particular a rat.

11. Holding device according to anyone of the preceding claims, **characterized in that** the beds (11a, 11b, 11c, 11d) each have a semicircular cross section.

12. Holding device according to anyone of the preceding, claims, **characterized in that**
holding bars or stopper elements (17a, 17b, 17c, 17d) are provided, each having a notch (18a, 18b, 18c, 18d) for holding a tail (T) of the small animal (A) while lying on its bed (11a, 11b, 11c, 11d).

13. Holding device according to anyone of the preceding claims, **characterized in that** the holder elements (12a, 12b, 12c) each have a slot or slit (19a, 19b, 19c, 19d) for receiving and holding inlet tubes for leading anesthetization gas to a gas mask for the respective small animal (A).

## Patentansprüche

1. Haltevorrichtung (10) zum gleichzeitigen Halten mehrerer Kleintiere (A), umfassend
mindestens drei Liegeflächen (11a, 11b, 11c, 11d), jeweils für ein Kleintier (A), wobei jede Liegefläche (11a, 11b, 11c, 11d) an einem entsprechenden Halteelement (12a, 12b, 12c) befestigt ist,
**dadurch gekennzeichnet**
**dass** die Haltevorrichtung (10) geometrisch so aufgebaut und mechanisch so ausgebildet ist, dass zwei der Halteelemente (12a, 12b) zusammen mit ihren jeweiligen Liegeflächen (11a, 11b) über ein drittes Halteelement (12c) bewegt werden können, ohne dabei die vertikale Ausrichtung der an den zwei Halteelementen (12a, 12b) befestigten Liegeflächen (11a, 11b) zu verändern, und dass die zwei Halteelemente (12a, 12b) durch eine Translationsbewegung oder durch eine Drehbewegung über das dritte Halteelement (12c) bewegt werden können.

2. Haltevorrichtung nach Anspruch 1, **dadurch gekennzeichnet, dass** die zwei Halteelemente (12a, 12b), die über das dritte Halteelement (12c) bewegt werden können, jeweils mit mindestens zwei Haltearmen (13a, 13a'; 13b, 13b') schwenkbar mit dem dritten Halteelement (12c) verbunden sind.

3. Haltevorrichtung nach einem der vorhergehenden Ansprüche, **dadurch gekennzeichnet, dass** die Haltevorrichtung (10) einen Verriegelungsmechanismus aufweist, der geometrisch so konstruiert und mechanisch so ausgebildet ist, dass zwei der Halteelemente (12a, 12b) in ihrer Bewegung verriegelt und in einer zusammengeklappten Position, in der mindestens eines des ersten und zweiten Halteelements (12a, 12b) über dem dritten Halteelement (12c) angeordnet ist, und in einer ausgeklappten Position, in der mindestens eines des ersten und zweiten Halteelements (12a, 12b) horizontal neben dem dritten Halteelement (12c) angeordnet ist, fixiert werden können.

4. Haltevorrichtung nach den Ansprüchen 2 und 3, **dadurch gekennzeichnet, dass** die Haltearme (13a, 13a'; 13b, 13b') der beiden Halteelemente (12a, 12b) Vertiefungen (14a, 14a'; 14b, 14b') aufweisen und das dritte Halteelement (12c) federbelastete Kugeln (15a, 15b) aufweist, die in die Vertiefungen (14a, 14a'; 14b, 14b') eingreifen können, so dass die zwei Halteelemente (12a, 12b) in der zusammengeklappten Position oder in der ausgeklappten Position relativ zum dritte Halteelement (12c) fixiert werden können.

5. Haltevorrichtung nach Anspruch 4, **dadurch gekennzeichnet, dass** die Vertiefungen (14a, 14a'; 14b, 14b') eine schlitzartige Struktur aufweisen.

6. Haltevorrichtung nach Anspruch 4 oder 5, **dadurch gekennzeichnet, dass** das dritte Halteelement (12c) zwei federbelastete Kugeln (15a, 15b) umfasst, die in der ausgeklappten bzw. in der zusammengeklappten Position in die Vertiefung der oberen Haltearme (13a, 13b) und der unteren Haltearme (13a', 13b') eingreifen.

7. Haltevorrichtung nach einem der vorhergehenden Ansprüche, **dadurch gekennzeichnet, dass** das erste und das zweite Halteelement (12a, 12b) jeweils an ihrer Unterseite eine flache Oberfläche (16a, 16b) aufweisen, so dass das erste und das zweite Halteelement (12a, 12b) in einer ausgeklappten Position stabil auf einer ebenen Arbeitsfläche, insbesondere auf einem Schreibtisch, stehen können, wobei mindestens eines des ersten und zweiten Halteelements (12a, 12b) horizontal neben dem dritten Halteelement (12c) angeordnet ist.

8. Haltevorrichtung nach einem der vorhergehenden Ansprüche, **dadurch gekennzeichnet, dass** die Haltevorrichtung (10) in einer vollständig zusammengeklappten Position, in der das erste und das zweite Halteelement (12a, 12b) beide über dem dritten Halteelement (12c) angeordnet sind, eine zylindrische Form mit kreisförmigem Umfang bildet.

9. Haltevorrichtung nach einem der Ansprüche 1 bis 8, **dadurch gekennzeichnet, dass** das dritte Halteelement (12c) zwei Liegeflächen (11c, 11d) für Kleintiere (A), insbesondere Mäuse, umfasst.

10. Haltevorrichtung nach einem der Ansprüche 1 bis 8, **dadurch gekennzeichnet, dass** das dritte Halteelement (12c) eine Liegefläche (11c) für ein Kleintier (A), insbesondere eine Ratte, umfasst.

11. Haltevorrichtung nach einem der vorhergehenden Ansprüche, **dadurch gekennzeichnet, dass** die Liegeflächen (11a, 11b, 11c, 11d) jeweils einen halbkreisförmigen Querschnitt aufweisen.

12. Haltevorrichtung nach einem der vorhergehenden Ansprüche, **dadurch gekennzeichnet, dass** Haltebügel oder Anschlagelemente (17a, 17b, 17c, 17d) vorgesehen sind, die jeweils eine Aussparung (18a, 18b, 18c, 18d) zum Halten eines Schwanzes (T) des Kleintiers (A) aufweisen, während das Kleintier auf seiner Liegefläche (11a, 11b, 11c, 11d) liegt.

13. Haltevorrichtung nach einem der vorhergehenden Ansprüche, **dadurch gekennzeichnet, dass** die Halteelemente (12a, 12b, 12c) jeweils einen Schlitz oder einen Spalt (19a, 19b, 19c, 19d) zur Aufnahme und zum Halten von Einlassschläuchen aufweisen, um Anästhesiegas zu einer Gasmaske für das jeweilige Kleintier (A) zu leiten.

## Revendications

1. Dispositif de maintien (10) pour maintenir simultanément plusieurs petits animaux (A) comprenant au moins trois lits (11a, 11b, 11c, 11d) chacun pour un petit animal (A), chaque lit (11a, 11b, 11c, 11d) étant fixé à un élément de support correspondant (12a, 12b, 12c),
**caractérisé**
**en ce que** le dispositif de maintien (10) est construit géométriquement et configuré mécaniquement de telle sorte que deux des éléments de support (12a, 12b) peuvent être déplacés, avec leurs lits respectifs (11a, 11b), au-dessus d'un troisième élément de support (12c) sans modifier ainsi l'orientation verticale des lits (11a, 11b) qui sont fixés aux deux éléments de support (12a, 12b), et **en ce que** les deux éléments de support (12a, 12b) peuvent être déplacés au-dessus du troisième élément de support (12c) par un mouvement de translation ou par un mouvement de rotation.

2. Dispositif de maintien selon la revendication 1, **caractérisé en ce que** les deux éléments de support (12a, 12b) qui peuvent être déplacés au-dessus du troisième élément de support (12c) sont reliés pivotants audit troisième élément de support (12c) chacun avec au moins deux bras de support (13a, 13a' ; 13b,13b').

3. Dispositif de maintien selon l'une quelconque des revendications précédentes, **caractérisé en ce que** le dispositif de maintien (10) comprend un mécanisme de verrouillage qui est construit géométriquement et configuré mécaniquement de telle sorte que deux des éléments de support (12a, 12b) lors de leur mouvement peuvent être verrouillés et fixés dans une position repliée avec au moins l'un du premier et du second élément de support (12a, 12b) étant placé au-dessus du troisième élément de support (12c) et dans une position dépliée avec au moins l'un du premier et du second élément de support (12a, 12b) étant placé horizontalement à côté du troisième élément de support (12c).

4. Dispositif de maintien selon les revendications 2 et 3, **caractérisé en ce que** les bras de support (13a,13a' ; 13b,13b') des deux éléments de support (12a, 12b) présentent des évidements (14a,14a'; 14b,14b') et le troisième élément de support (12c) présente des billes à ressort (15a, 15b) qui peuvent s'engager dans les évidements (14a,14a' ; 14b,14b') de sorte que les deux éléments de support (12a, 12b) peuvent être fixés par rapport au troisième élément de support (12c) en position repliée ou en position dépliée.

5. Dispositif de maintien selon la revendication 4, **caractérisé en ce que** les évidements (14a,14a' ; 14b,14b') présentent une structure en forme de fente.

6. Dispositif de maintien selon la revendication 4 ou 5, **caractérisé en ce que** le troisième élément de support (12c) comprend deux billes à ressort (15a, 15b) qui s'engagent dans l'évidement des bras de support supérieurs (13a, 13b) et des bras de support inférieurs (13a', 13b') respectivement en position dépliée et en position repliée.

7. Dispositif de maintien selon l'une quelconque des revendications précédentes, **caractérisé en ce que** le premier et le second élément de support (12a, 12b) présentent chacun une surface plane (16a, 16b) à leur base de sorte que le premier et le second élément de support (12a, 12b) peuvent reposer de manière stable sur une surface de travail plane, en particulier sur un bureau, en position dépliée avec au moins l'un du premier et du second élément de support (12a, 12b) étant placé horizontalement à côté du troisième élément de support (12c).

8. Dispositif de maintien selon l'une quelconque des revendications précédentes, **caractérisé en ce qu'**en position complètement repliée avec le premier et le second élément de support (12a, 12b) étant tous deux placés au-dessus du troisième élément de support (12c), le dispositif de maintien (10) forme une forme cylindrique à circonférence circulaire.

9. Dispositif de maintien selon l'une quelconque des revendications 1 à 8, **caractérisé en ce que** le troisième élément de support (12c) comprend deux lits (11c, 11d) pour petits animaux (A), en particulier des souris.

10. Dispositif de maintien selon l'une quelconque des revendications 1 à 8, **caractérisé en ce que** le troisième élément de support (12c) comprend un lit (11c) pour un petit animal (A), en particulier un rat.

11. Dispositif de maintien selon l'une quelconque des revendications précédentes, **caractérisé en ce que** les lits (11a, 11b, 11c, 11d) présentent chacun une section transversale semi-circulaire.

12. Dispositif de maintien selon l'une quelconque des revendications précédentes, **caractérisé en ce que** des barres de maintien ou des éléments d'arrêt (17a, 17b, 17c, 17d) sont prévus, chacun présentant une encoche (18a, 18b, 18c, 18d) pour maintenir une queue (T) du petit animal (A) pendant qu'il est couché sur son lit (11a, 11b, 11c, 11d).

13. Dispositif de maintien selon l'une quelconque des revendications précédentes, **caractérisé en ce que** les éléments de support (12a, 12b, 12c) présentent chacun une fente ou une fente (19a, 19b, 19c, 19d) pour recevoir et maintenir des tubes d'admission pour amener du gaz d'anesthésie à un masque à gaz pour le petit animal (A) respectif.
